# EUROPÄISCHE PATENTSCHRIFT

(11) **EP 1 634 565 B1**
(45) Veröffentlichungstag und Bekanntmachung des Hinweises auf die Patenterteilung: **01.04.2009**
(21) Anmeldenummer: 05013753.8
(22) Anmeldetag: 25.06.2005
(51) Int. Cl.: A61K 8/04, A61K 8/92, A61Q 19/00

(54) **Peelingkörper für kosmetische Mittel**
Peeling body for cosmetic composition
Composition exfoliante pour le corps

(30) Priorität: 20.08.2004 DE 102004040329
(43) Veröffentlichungstag der Anmeldung: 15.03.2006
(73) Patentinhaber: Henkel AG & Co. KGaA, 40589 Düsseldorf (DE)
(72) Erfinder: Kampmann, Martina, 41352 Korschenbroich (DE)

(56) Entgegenhaltungen:
- EP-A- 0 412 865
- WO-A-98/11872
- WO-A-02/051376
- CH-A5- 678 488
- DE-A1- 10 210 449
- DE-A1- 19 958 521
- PATENT ABSTRACTS OF JAPAN Bd. 011, Nr. 185 (C-428), 13. Juni 1987 (1987-06-13) & JP 62 010009 A (NONOGAWA SHOJI:KK), 19. Januar 1987 (1987-01-19)
- PATENT ABSTRACTS OF JAPAN Bd. 2003, Nr. 12, 5. Dezember 2003 (2003-12-05) & JP 2004 189612 A (MANDOM CORP), 8. Juli 2004 (2004-07-08)
- "Römpp Chemie-Lexikon, 9. Auflage, Band 3, Seite 2100" 1995, GEORG THIEME VERLAG , STUTTGART
- "Römpps Chemie-Lexikon, 9. Auflage, Band 1, Seite 594" 1995, GEORG THIEME VERLAG , STUTTGART

## Beschreibung

Die Erfindung betrifft Peelingkörper für kosmetische Mittel, deren Verwendung in kosmetischen Mitteln und diese enthaltende kosmetische Mittel.

Kosmetische Peeling-Produkte enthalten häufig abrasiv wirkende Teilchen wie Polyethylenpulver, Walnussschalenpulver oder Aprikosen- oder Mandelkem-Pulver. Diese Pulver können bei der Anwendung zur Hautreizungen führen, da sie sich bei der Hautbehandlung selbst nicht verändern und häufig zumindest teilweise auf der Haut verbleiben. Aufgrund dieser Reizwirkung können entsprechende Peeling-Formulierungen in der Regel nicht täglich angewendet werden, sondern es müssen Zeiträume ohne Anwendung verbleiben, in denen sich die Haut regenerieren kann. Darüber hinaus ist es häufig nach der Anwendung derartiger Produkte notwendig, Hautpflegecremes auf die behandelte Haut aufzutragen, um die durch die Behandlung angegriffene Haut zu pflegen und feucht zu halten.

Es besteht daher weiterhin Nachfrage nach verbesserten Peeling-Produkten, die die Nachteile der bekannten Produkte vermeiden.

Die US 5,665,687 betrifft eine Reinigungszusammensetzung, die Lipid-Teilchen enthält. Die Lipid-Teilchen sind dabei mit einem Reinigungsmittel gefüllt. Die Lipid-Teilchen leiten sich dabei von halbsynthetischen Glyceriden, natürlichen oder synthetischen Fetten ab. Insbesondere handelt es sich um lineare gesättigte Fettsäuretriglyceride. Natürliche Fette sind beispielsweise Pflanzenfette. Es können auch Silikonwachse eingesetzt werden. Als Reinigungsmittel wird in den Lipid-Teilchen beispielsweise Weizenmehl eingesetzt. Auch der Einsatz von Tonen oder Ölen ist möglich. Die festen, nicht-hydrophilen Lipidteilchen haben einen Schmelzpunkt unterhalb von 50 °C. Beim Massieren der Haut mit einer Zusammensetzung, die die Lipidteilchen enthält, wird ein Peeling-Effekt erreicht, durch den tote Hautzellen von der Haut entfernt werden. Bei diesem Vorgang schmelzen die Teilchen, ohne dass es zu einer Reizwirkung kommt. Ferner wird eine Hautpflegewirkung durch die Auswahl der Inhaltsstoffe der kosmetischen Emulsionen, insbesondere Öle und Aktivstoffe, erreicht.

Die EP-A-0 412 865 betrifft ein wässriges Gel, das Kügelchen aus einem festen nicht hydrophilen Lipid suspendiert enthält. Das Gel ist dabei insbesondere eine Emulsion des Öl-in-Wasser-Typs. Durch Einsatz von Lipid-Teilchen in den Gelen werden die austrocknende Wirkung der Gele und der Mangel an Behaglichkeit bei der Anwendung vermindert. Die Lipid-Teilchen weisen wiederum einen Schmelzpunkt von unter 50 °C auf, wobei halbsynthetische Glyceride, natürliche und synthetische Fette eingesetzt werden können.

Die WO 03/074021 betrifft eine isotrope flüssige Reinigungszusammensetzung, die neben einem Tensid und einem Verdicker auch Organogelteilchen enthält, die wiederum eine Pflegekomponente enthalten.

JP 62 010009 A und JP 2004 189612 A beschreiben die Verwendung von Peelingkörpern aus hydriertem Jojoba- Öl eines Teilchendurchmessers von 0,1 bis 2.0 mm bzw. aus Carnauba-Wachs eines Teilchendurchmessers von 0,05 bis 1.0 mm.

Die bekannten Mittel sind aufwendig in der Herstellung und nur in Form spezieller Formulierungen darstellbar. Aufgabe der vorliegenden Erfindung ist die Bereitstellung von Peelingkörpern, die eine verminderte Hautreizung zeigen, neben einer Massage- und Reinigungswirkung auch eine Pflegewirkung zeigen und für den Anwender den Endpunkt der Anwendung des Mittels anzeigen. Vorzugsweise soll der Peelingkörper auch eine schaumstärkende Wirkung in tensidischen Produkten, verbunden mit einer besseren Pflegewirkung, aufweisen. Zudem sollen die Peelingkörper unaufwendig herstellbar sein.

Die Aufgaben werden erfindungsgemäß gelöst durch Verwendung von gesättigten oder ungesättigten Fettsäuren mit mindestens 18 C-Atomen, deren Mono- oder Diglyceriden oder Mischungen davon, in Teilchenform mit einem mittleren Teilchendurchmesser im Bereich von 0,05 bis 2 mm, in kosmetischen Mitteln.

Die Aufgaben werden ferner erfindungsgemäß gelöst durch ein kosmetisches Mittel, enthaltend, bezogen auf das gesamte Mittel 0,05 bis 10 Gew.-% an gesättigten oder ungesättigten Fettsäuren mit mindestens 18 C-Atomen, deren Mono- oder Diglyceriden oder Mischungen davon, in Teilchenform mit einem mittleren Teilchendurchmesser von 0,05 bis 2 mm.

Die Aufgaben werden ferner gelöst durch ein Teilchen mit einem mittleren Teilchendurchmesser im Bereich von 0,05 bis 2 mm, enthaltend, bezogen auf die Teilchen, mindestens 50 Gew.-% gesättigte oder ungesättigte Fettsäuren mit mindestens 18 C-Atomen, deren Mono- oder Diglyceride oder Mischungen davon.

Es wurde erfindungsgemäß gefunden, dass Peelingkörper aus gesättigten oder ungesättigten Fettsäuren mit mindestens 18 C-Atomen, deren Mono- oder Diglyceriden oder Mischungen davon, eine vorteilhafte Massage und Reinigungswirkung in kosmetischen Formulierungen haben und darüber hinaus eine pflegende Wirkung entfalten. Zudem wirken die Teilchen schaumstabilisierend in tensidischen Produkten.

Es ist erfindungsgemäß möglich, durch die erfindungsgemäßen Peelingkörper sowohl die teilweise kostenaufwendigeren üblichen Peelingkörper zu ersetzen, wobei gleichzeitig in kosmetischen Zusammensetzungen der Anteil an pflegenden Komponenten vermindert werden kann, da die erfindungsgemäßen Peelingkörper bereits eine Pflegewirkung entfalten.

Die erfindungsgemäß eingesetzten Teilchen weisen einen mittleren Teilchendurchmesser im Bereich von 0,05 bis 2 mm, vorzugsweise 0,1 bis 1 mm auf. Je nach Anwendung, beispielsweise zur Behandlung der Haut im Gesicht oder an den Füssen, kann die Teilchengröße in den genannten Bereichen frei gewählt werden. Für ein Mittel zur Gesichtsbehandlung liegt die mittlere Teilchengröße vorzugsweise im Bereich von 0,1 bis 0,5 mm. Für ein Mittel zur Hautbehandlung an den Füssen kann die mittlere Teilchengröße auch in einem höheren Bereich von 0,5 bis 2 mm liegen.

Die Teilchen können dabei jede geeignete Geometrie aufweisen, die sich aus ihrem Herstellverfahren ableiten kann. Sofern die Teilchen durch Schneiden oder Brechen oder Mahlen hergestellt werden, wird häufig eine geeckte Struktur erhalten, während beim Erstarren aus Flüssigkeiten, insbesondere durch Sprühtrocknungsprozesse, kugelförmige oder elliptische Teilchen und damit im Wesentlichen eckenfreie Teilchen erhalten werden. Vorzugsweise werden elliptische bis kugelförmige Teilchen eingesetzt. Verfahren zur Herstellung der Teilchen, insbesondere durch Sprühtrocknungsverfahren, sind dem Fachmann bekannt. Die Herstellung kann auch durch Vermahlen bei tiefen Temperaturen erfolgen, wie es beispielsweise in US 5,665,687, Spalte 4, Zeilen 32 bis 50 beschrieben ist. Es wird insbesondere ein gesprühtes Produkt eingesetzt. Die Teilchen schmelzen oder erweichen vorzugsweise im Temperaturbereich von 20 bis 50 °C, insbesondere 30 bis 50 °C. Besonders bevorzugt liegt der Schmelzpunkt der Teilchen im Bereich von 30 bis 50 °C, insbesondere 35 bis 45 °C.

Dem Fachmann sind dabei entsprechende Teilchen aus gesättigten oder ungesättigten Fettsäuren oder Mono- oder Diglyceriden oder Mischungen davon bekannt. Bevorzugt werden gesättigte Fettsäuren eingesetzt, die mindestens 18 C-Atome aufweisen, ungesättigte Fettsäuren, die mindestens 20 C-Atome aufweisen oder Mono- oder Diglyceride von Fettsäuren mit mindestens 22 C-Atomen.

Besonders bevorzugt werden gesättigte C₁₈₋₃₀-Fettsäuren, insbesondere gesättigte C₁₈₋₂₄-Fettsäuren eingesetzt. Es können ebenfalls besonders bevorzugt ungesättigte C₂₀₋₃₀-Fettsäuren, insbesondere C₂₀₋₂₄-Fettsäuren eingesetzt werden. Als Mono- oder Diglyceride vorliegender Fettsäuren werden vorzugsweise solche mit 22 bis 30, insbesondere 22 bis 24 C-Atomen eingesetzt.

Es können auch Mischungen der genannten Verbindungen eingesetzt werden, sofern sie einen geeigneten Schmelz- oder Erweichungspunkt aufweisen.

Fettsäurepartialglyceride sind in der Regel technische Gemische von Monoglyceriden und Diglyceriden. Herstellungsbedingt können dabei auch noch geringe Mengen Triglyceride enthalten sein, deren Anteil aber vorzugsweise weniger als 10 Gew.-%, besonders bevorzugt weniger als 3 Gew.-%, insbesondere weniger als 1 Gew.-% beträgt. Zwischen Acylrest und Glycerin-Grundkörper können auch noch (Poly)ethylenoxid-Einheiten eingebaut sein. Bevorzugt liegen jedoch keine solchen zusätzlichen von Ethylenoxyd abgeleiteten Bausteine vor. Typische Beispiele sind Mono- und/oder Diglyceride auf Basis der Fettsäuren, die unter den Esterölen aufgeführt sind.

Die erfindungsgemäß eingesetzten Teilchen enthalten dabei, bezogen auf die Teilchen, mindestens 50 Gew.-% der vorstehend genannten Verbindungen. Besonders bevorzugt enthalten sie mindestens 85 Gew.-%, insbesondere mindestens 95 Gew.-% der vorstehend genannten Verbindungen. Speziell bestehen die Teilchen aus den vorstehend genannten Verbindungen oder deren Gemischen, und es liegen keine weiteren Inhaltsstoffe in den Teilchen vor.

Die Teilchengröße kann mittels bekannter Verfahren bestimmt werden, beispielsweise mikroskopisch, durch Sieben oder durch Klassieren.

Die erfindungsgemäß eingesetzten Teilchen können Hilfsstoffe enthalten, beispielsweise Duftstoffe, Konservieningsstoffe, Farbstoffe, Pigmente, Antioxidantien usw. Sie können auch hydrophile oder liphophile Aktivstoffe wie Polyole, Ceramide und ähnliche Verbindungen enthalten. Ferner können Vitamine in die Teilchen eingearbeitet werden. Besonders bevorzugt werden die erfindungsgemäßen Teilchen jedoch ohne Hilfsstoffe hergestellt und eingesetzt.

Die Teilchen werden erfindungsgemäß insbesondere als pflegende Peelingkörper in den kosmetischen Mitteln eingesetzt. Dabei dienen die kosmetischen Mittel insbesondere zur kosmetischen Behandlung, beispielsweise Reinigung, Peeling und/oder Pflege, der menschlichen Haut. Beim Einreiben der Haut mit einem erfindungsgemäßen Mittel, das die erfindungsgemäßen Peelingkörper enthält, wird zunächst ein Massage- und Reinigungseffekt erzielt, bei dem tote Hautzellen von der Haut abgelöst werden. Durch die Behandlung mit den erfindungsgemäßen Peelingkörpern erwärmen sich diese über ihren Schmelzpunkt, so dass zum Abschluss der Behandlung ein pflegender Effekt durch die Fettsäuren oder Mono- oder Diglyceride davon erreicht wird. Ferner wird dem Benutzer der Endpunkt der Behandlung durch das Aufschmelzen der Peelingkörper angezeigt. Das Hautgefühl der aufgetragenen Mittel verändert sich zu diesem Zeitpunkt, so dass der Anwender ein Signal zur Beendigung der Anwendung erhält. Die Peelingkörper verschwinden damit während der Anwendung und dienen damit zunächst als Peelingkörper und danach als pflegende Substanz, die auf der Haut verbleiben kann. Die erfindungsgemäßen Mittel, die die Peelingkörper enthalten, haben damit eine Reinigungswirkung, eine abrasive Wirkung und eine Pflegewirkung. Vorzugsweise können mit den erfindungsgemäßen Peelingkörpern auch Talgverhärtungen in den Poren (Black spots) gelöst und entfernt werden.

Ferner zeigen die erfindungsgemäßen Peelingkörper vorzugsweise eine schaumstärkende oder schaumstabilisierende Wirkung in tensidischen Produkten. Hierdurch kann die Anwendungsdauer des Produktes verlängert werden, da ein Einmassieren mit einem geschäumten Produkt so lange fortgeführt werden kann, bis die Schaummenge abnimmt.

Die erfindungsgemäßen Peelingkörper bzw. -teilchen werden vorzugsweise in kosmetische Mittel in einer Menge von 0,05 bis 10 Gew.-%, besonders bevorzugt 0,1 bis 5 Gew.-%, insbesondere 0,5 bis 2,5 Gew.-% eingebracht, bezogen auf das gesamte Mittel. Das kosmetische Mittel liegt dabei vorzugsweise in Form von tensidhaltigen wässrigen Lösungen oder in Form von Emulsionen vor.

Die erfindungsgemäßen Peelingkörper können auch in feste oder flüssige Seifen, Syndet-Seifen oder Halbsyndet-Seifen integriert werden.

Tensidhaltige wässrige Lösungen können dabei Wasser als ausschließliches Lösungsmittel oder Wasser in Mischung mit weiteren hautverträglichen Lösungsmitteln, insbesondere Alkoholen wie Ethanol, enthalten. Besonders bevorzugt sind wässrige Systeme, die bis zu 50 Gew.-% Wasser enthalten. Die erfindungsgemäßen kosmetischen Mittel können dabei in Form einer Creme, Lotion, Lösung, Emulsion, Schüttelmixtur oder in Form eines Gels vorliegen. Geeignete Grundformulierungen, insbesondere für Peeling-Produkte zur Behandlung der Gesichtshaut, sind bekannt. Für mögliche weitere Inhaltsstoffe kann beispielsweise auf US 5,665,687 bzw. EP-A-0 692 236 sowie EP-A-0 412 865 verwiesen werden.

Insbesondere enthalten die erfindungsgemäßen kosmetischen Mittel neben den erfindungsgemäßen Peelingkörpern, z. B. 0,1 bis 40 Gew.-%, Tenside und/oder, z. B. 0,1 bis 25 Gew.-%, Emulgatoren.

Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind. Weitergehende Definitionen und Eigenschaften von Tensiden finden sich in "H.-D. Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (VI), 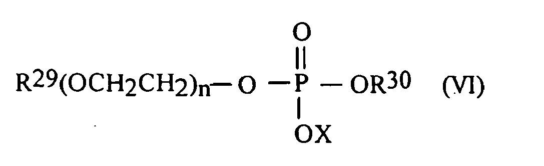 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- sulfatierte Fettsäurealkylenglykolester der Formel (VII)
- R³⁵CO(AlkO)ₙSO₃M (VII) in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (VIII), 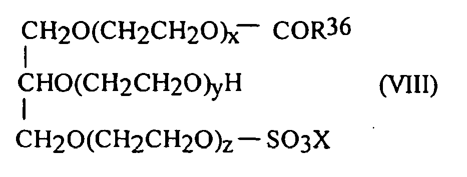 in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (VIII) eingesetzt, in der R³⁶CO für einen linearen Acylrest mit 8 bis 18 Kohlenstoffatomen steht. Monoglyceridsulfate und Monoglyceridethersulfate werden beispielsweise in der EP-B1 0 561 825, der EP-B1 0 561 999, der DE-A1 42 04 700 oder von A.K. Biswas et al. in J.Am.Oil.Chem.Soc. 37, 171 (1960) und F.U. Ahmed in J.Am.Oil.Chem.Soc. 67, 8 (1990) beschrieben.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die so genannten Betaine wie die N-Alkyl-N,N-dimethylammonium-glycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ -Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel R³⁷CO-(OCH₂CHR³⁸)_{w}OR³⁹, (IX), in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im Wesentlichen aus C₈- und C₁₀-Alkylgruppen,
- im Wesentlichen aus C₁₂- und C₁₄-Alkylgruppen,
- im Wesentlichen aus C₈- bis C₁₆-Alkylgruppen oder
- im Wesentlichen aus C₁₂- bis C₁₆-Alkylgruppen oder
- im Wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung so genannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Diese Tenside werden in Mengen von 0,1 - 45 Gew.%, bevorzugt 1 - 30 Gew.% und ganz besonders bevorzugt von 1 -15 Gew.%, bezogen auf das gesamte Mittel, eingesetzt.

In einer bevorzugten Ausführungsform können nicht-ionische, zwitterionische und/oder amphotere Tenside sowie deren Mischungen bevorzugt sein.

Erfindungsgemäß einsetzbar sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Zu den erfindungsgemäß eingesetzten Tensid und/oder Emulgatorkomponenten sind auch die wie folgt definierten Emulgatoren zu zählen: Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994". Erfindungsgemäß verwendbare Emulgatoren sind beispielsweise:
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die so genannten Mykosterine, isoliert.
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Mittel mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Unter den genannten Emulgatoren-Typen können die Emulgatoren, welche kein Ethylenoxid und/oder Propylenoxid im Molekül enthalten, ganz besonders bevorzugt sein.

In einer bevorzugten Ausführungsform der erfindungsgemäßen Lehre kann die Wirkung der Mittel mit Polymeren weiter gesteigert werden. Unter Polymeren sind sowohl natürliche als auch synthetische Polymere, welche anionisch, kationisch, amphoter geladen oder nichtionisch sein können, zu verstehen.

Unter kationischen Polymeren sind Polymere zu verstehen, welche in der Haupt- und/oder Seitenkette Gruppen aufweisen, welche "temporär" oder "permanent" kationisch sein können. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (III), 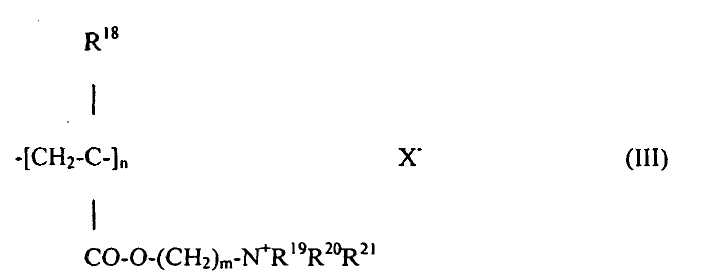 in der R¹⁸ = -H oder -CH₃ ist, R¹⁹, R²⁰ und R²¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im Wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁸ steht für eine Methylgruppe
- R¹⁹, R²⁰ und R²¹ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquatemium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecylpolyoxypropylen-polyoxyethylenether (INCI-Bezeichnung: PPG-1 -Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Coming; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol, sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Mittel muss das Chitosan in die Salzform überführt werden. Dies kann durch Auflösen in verdünnten wässrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Bei den anionischen Polymeren, welche die Wirkung des erfindungsgemäßen Mittels unterstützen können, handelt es sich um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppenhaltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel^{®}305 der Firma SEPPIC enthalten.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter der Marke Carbopol^{®} im Handel erhältlich.

Copolymere aus Maleinsäureanhydrid und Methylvinylether, insbesondere solche mit Vernetzungen, sind ebenfalls einsetzbare Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere amphotere Polymere als Bestandteil eingesetzt werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefasst, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im Wesentlichen zusammensetzen aus
(a) Monomeren mit quartären Ammoniumgruppen der allgemeinen Formel (IV),

   R²²-CH=CR²³-CO-Z-(CₙH₂ₙ)-N⁽⁺⁾R²⁴R²⁵R²⁶ A⁽⁻⁾ (IV)

   in der R²² und R²³ unabhängig voneinander stehen für Wasserstoff oder eine Methylgruppe und R²⁴, R²⁵ und R²⁶ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist und
(b) monomeren Carbonsäuren der allgemeinen Formel (V),

   R²⁷-CH=CR²⁸-COOH (V)

   in denen R²⁷ und R²⁸ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R²⁴, R²⁵ und R²⁶ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer dritten Variante weiterhin nichtionogene Polymere enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone gemäß der EP 0612759 B1.

Es ist erfindungsgemäß auch möglich, dass die Mittel mehrere, insbesondere zwei verschiedene Polymere gleicher Ladung und/oder jeweils ein ionisches und ein amphoteres und/oder nicht ionisches Polymer enthalten.

Ein solches Verfahren ist z.B. aus EP 466 986 B1 bekannt. Geeignete Polymerisate sind z.B. Polycarbonate, Polyurethane, Polyacrylate, Polyolefine, Polyester oder Polyamide. Besonders geeignet sind solche sphärischen Polymerpulver, deren Primärpartikeldurchmesser unter 1 µm liegt. Solche Produkte auf Basis eines Polymethacrylat-Copolymers sind z.B. unter dem Warenzeichen Polytrap^{®}Q5-6603 (Dow Coming) im Handel. Andere Polymerpulver, z.B. auf Basis von Polyamiden (Nylon 6, Nylon 12) sind mit einer Teilchengröße von 2 - 10 µm (90 %) und einer spezifischen Oberfläche von ca. 10 m²/g unter der Handelsbezeichnung Orgasol^{®} 2002 DU Nat Cos (Atochem S.A., Paris) erhältlich. Weitere sphärische Polymerpulver, die für den erfindungsgemäßen Zweck geeignet sind, sind z.B. die Polymethacrylate (Micropearl M) von SEPPIC oder (Plastic Powder A) von NIKKOL, die Styrol-Divinylbenzol-Copolymeren (Plastic Powder FP) von NIKKOL, die Polyethylen- und Polypropylen-Pulver (ACCUREL EP 400) von AKZO, oder auch Silikonpolymere (Silicone Powder X2-1605) von Dow Coming oder auch sphärische Cellulosepulver.

Die Polymere sind in den erfindungsgemäß verwendeten Mitteln bevorzugt in Mengen von 0,01 bis 10 Gew.-%, bezogen auf das gesamte Mittel, enthalten. Mengen von 0,1 bis 5, insbesondere von 0,1 bis 3 Gew.-%, sind besonders bevorzugt.

In einer weiteren Ausführungsform können die erfindungsgemäßen Mittel Proteinhydrolysate und deren Derivate enthalten. Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden.

In einer weiteren bevorzugten Ausführungsform der Erfindung kann die Wirkung des erfindungsgemäßen Mittels durch zusätzliche Fettstoffe weiter optimiert werden. Unter Fettstoffen sind zu verstehen Fettsäuren, Fettalkohole, natürliche und synthetische Wachse, welche sowohl in fester Form als auch flüssig in wässriger Dispersion vorliegen können, und natürliche und synthetische kosmetische Ölkomponenten zu verstehen.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure. Die Beispiele sind teilweise auch für die Peelingkörper gültig.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1 - 5 Gew.% sind (ohne die Peelingkörper gerechnet).

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnussöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die pflegende Wirkung des erfindungsgemäßen Peelingkörpers steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Diisotridecylacelaat sowie Diolester wie Ethylenglykoldioleat, Ethylenglykol-di-isotridecanoat, Propylenglykol-di(2-ethylhexanoat), Propylenglykoldi-isostearat, Propylenglykoldipelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12 oder Cutina^{®} MD.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 6 - 45 Gew.-%, bezogen auf das gesamte Mittel. Mengen von 10-35 Gew.-% sind erfindungsgemäß bevorzugt.

Die Mittel können auch Hydroxycarbonsäureester enthalten. Bevorzugte Hydroxycarbonsäureester sind Vollester der Glycolsäure, Milchsäure, Äpfelsäure, Weinsäure oder Citronensäure. Weitere grundsätzlich geeignete Hydroxycarbonsäureester sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Die Einsatzmenge der Hydroxycarbonsäureester beträgt dabei 0,1 - 15 Gew.% bezogen auf das Mittel, bevorzugt 0,1 - 10 Gew.% und ganz besonders bevorzugt 0,1 - 5 Gew.%.

Ebenfalls als vorteilhaft hat sich die Kombination der Peelingkörper mit Vitaminen, Provitaminen und Vitaminvorstufen sowie deren Derivaten erwiesen.
Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure und Panthenol) und dessen Vorstufe Pantolacton. Im Rahmen dieser Gruppe wird bevorzugt das Panthenol eingesetzt. Erfindungsgemäß einsetzbare Derivate des Panthenols sind insbesondere die Ester und Ether des Panthenols, kationisch derivatisierte Panthenole sowie Pantolacton. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Mittel Vitamine, Provitamine und Vitaminvorstufen aus den Gruppen A, B, E und H.

Panthenol und seine Derivate sowie Nicotinsäureamid und Biotin sind besonders bevorzugt.

Schließlich lässt sich die Wirkung der Mittel auch durch den kombinierten Einsatz mit Pflanzenextrakten steigern.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Neben dem erfindungsgemäß zwingend erforderlichen Peelingkörper und den weiteren, oben genannten bevorzugten Komponenten können diese Zubereitungen prinzipiell alle weiteren, dem Fachmann für solche kosmetischen Mittel bekannten Komponenten enthalten.

Weitere Wirk-, Hilfs- und Zusatzstoffe sind beispielsweise:
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Parfümöle,
- Dimethylisosorbid,
- Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- weitere Substanzen zur Einstellung des pH-Wertes, wie beispielsweise α- und β-Hydroxycarbonsäuren,
- Wirkstoffe wie Allantoin und Bisabolol,
- Cholesterin,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Treibmittel wie Propan-Butan-Gemische, N₂O, Dimethylether, CO₂ und Luft,
- Antioxidantien,
- Desoxyzucker,
- Pflanzenglycoside,
- Polysaccharide wie Fucose oder Rhamnose.

Bezüglich weiterer fakultativer Komponenten sowie die eingesetzten Mengen dieser Komponenten wird ausdrücklich auf die dem Fachmann bekannten einschlägigen Handbücher, z. B. die Monographie von Kh. Schrader, Grundlagen und Rezepturen der Kosmetika, 2. Auflage, Hüthig Buch Verlag, Heidelberg, 1989, verwiesen.

Als Konfektionierung der Mittel sind beispielsweise Seifen, Syndet-Seifen, Halbsyndet-Seifen, Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, grobe, instabile, ein oder mehrphasige Schüttelmixturen und Gele geeignet. Diese werden in der Regel auf wässriger oder wässrig-alkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wässrig alkoholischen Basis in einem Gewichtsverhältnis von 1 : 10 bis 10 : 1 vorliegen. Wasser sowie wässrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2 - 11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure oder Base verwendet werden. Üblicherweise werden als Säuren Genuss-Säuren verwendet. Unter Genuss-Säuren werden solche Säuren verstanden, die im Rahmen der üblichen Nahrungsaufnahme aufgenommen werden und positive Auswirkungen auf den menschlichen Organismus haben. Genuss-Säuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Monoethanolamin, Triethanolamin sowie N,N,N',N'-Tetrakis-(2-hydroxypropyl)-ethylendiamin.

Bei den erfindungsgemäßen Mitteln handelt es sich insbesondere um Rinse-Off-Mittel. Hierunter werden Mittel verstanden, die zunächst auf die Haut aufgebracht werden und danach nach einem Zeitraum von typischerweise 1 bis 15 Minuten mit Hilfe von Wasser oder einer wässrigen Lösung wieder von der Haut abgespült werden. Es handelt sich bei den erfindungsgemäßen Mitteln insbesondere um reinigende Mittel für die Haut, speziell um Gesichtsreiniger.

Die Erfindung wird durch die nachstehenden Beispiele näher erläutert.

Alle Mengenangaben sind, soweit nicht anders vermerkt, Gewichtsteile.

### 1. Feuchtigkeitscreme mit Vitamin E

| Bestandteil | Chem. Bezeichnung | INCI - Deklaration | Gew.% |
|---|---|---|---|
| EMULGADE^{®} PL 68/50 | Gemisch Alkylpolyglycosid und Cetylstearylalkohol | aus Cetearyl Glucoside (and) Cetearyl Alcohol | 5,0 |
| LANETTE^{®} E Pulver (Cognis) | Natriumcetylstearylsulfat | Sodium Cetearyl Sulfate | 0,25 |
| Glycerinmonostearat | mit einem Teilchendurchmesser von 1,5 mm | Glyceryl Stearate | 2,0 |
| MYRITOL 312 ^{®} (Cognis) | Capryl / Caprinsäuretriglycerid | Caprylic/Capric Triglyceride | 5,0 |
| CETIOL^{®} LC (Cognis) | Capryl / Caprinsäureester von gesättigten Fettalkoholen C 12 - C 18 | Coco- Caprylate/Caprate | 5,0 |
| EUTANOL^{®} G 16 (Cognis) | 2-Hexyldecanol (Guerbet-Alkohol) | Hexyldecanol | 2,0 |
| COPHEROL^{®} F 1300 (Cognis) | RRR-(α)-Tocopherol | Tocopherol | 1,0 |
| Wacker Siliconoil AK 350 (Wacker) | | Dimethicone | 0,5 |
| Valeranon | | | 0,5 |
| Glycerin 86% | | | 3,0 |
| D-Panthenol USP | | | 0,5 |
| Wasser | | | ad 100 |
| | | | |
| Viskosität (mPas), Brook.RVF, 23°C, Sp.TE, 4 Upm, mit 150000 Helipath | | | |

### 2. Allzweckcreme

| Bestandteil | Chem. Bezeichnung | INCI - Bezeichnung | Gew.% |
|---|---|---|---|
| DEHYMULS^{®} PGPH (Cognis) | Polyglycerinpoly-12-hydroxystearat | Polyglycerylpoly-12 hydroxystearate | 4,5 |
| MYRITOL^{®} 331 (Cognis) | | Cocoglycerides | 5,0 |
| CETIOL^{®} OE (Cognis) | Di-n-octyl Ether | Dicaprylyl Ether | 5,0 |
| Kamillenextrakt | | | 1,0 |
| Panthenol | | | 0,5 |
| Behensäure (Teilchengröße 1 mm) | | | 1,0 |
| Zinkstearat (Bärlocher) | | Zinestearate | 1,0 |
| Glycerin (86 %) | | | 5,0 |
| MgSO₄ . 7 H₂O | | | 0,5 |
| Wasser | | | ad 100 |
| Viskosität (mPas), Brookfield RVF, 23°C, Spindel TE, 4 Upm, mit Helipath | | ca. 200000 | |

### 3. Reichhaltige W/O Creme

| Bestandteil | Chem. Bezeichnung | INCI - Bezeichnung | Gew.% |
|---|---|---|---|
| DEHYMULS^{®} PGPH (Cognis) | Polyglycerinpoly-12-hydroxystearat | Polyglycerylpoly-12 hydroxystearate | 3,0 |
| LAMEFORM^{®} TGI (Cognis) | Triglycerindiisostearat | Polyglyceryl-3 Diisostearate | 3,0 |
| Bienenwachs 8100 (Fa. Kahl & Co.) | Bienenwachs | Cera Alba | 3,0 |
| Zincum^{®} N 29 (Fa. Bärlocher) | Zinkstearat | Zinc Stearate | 1,0 |
| CETIOL^{®} OE (Cognis) | Di-n-octyl Ether | Dicaprylyl Ether | 3,0 |
| CETIOL^{®} LC (Cognis) | Capryl / Caprinsäureester von gesättigten Fettalkoholen C 12 - C18 | Coco Caprylate/Caprate | 6,0 |
| MYRITOL^{®} 312 (Cognis) | Capryl / Caprinsäuretriglycerid | Caprylic/Capric Triglyceride | 8,0 |
| Almond Oil | Mandelöl | Almond Oil | 8,0 |
| COPHEROL^{®} F 1300 (Cognis) | RRR-(α)-Tocopherol | Tocopherol | 1,0 |
| Stearinsäure (1 mm Teilchengröße) | | | 2,0 |
| Glycerin | | | 5,0 |
| MgSO₄ x 7H₂O | | | 1,0 |
| Wasser | | | ad 100 |
| | | | |
| Viskosität (mPas) / Brookfield, RVF, 23°C, Spindel TE, 4 Upm, mit Helipath | | 150000 | |

### 4. Maske

| Bestandteil | Chem. Bezeichnung | INCI - Bezeichnung | Gew.% |
|---|---|---|---|
| DEHYQUART^{®} F 75 (Cognis) | Mischung aus Esterquat und Fettalkohol | Distearoylethyl Hydroxyethylmonium Methosulfate (and) Cetearyl Alcohol | 3,0 |
| LANETTE^{®} O (Cognis) | Cetylstearylalkohol | Cetearyl Alcohol | 4,0 |
| Glycerinmonostearat | Teilchengröße 0,5 mm | Glyceryl Stearate | 1,0 |
| EUMULGIN^{®} B 2 (Cognis) | Polyoxyethylen-20- Cetylstearylalkohol | Ceteareth-20 | 1,5 |
| NUTRILAN^{®} KERATIN W (Cognis) | Partialhydrolysat aus Keratin (ca. 20%) | Hydrolyzed Keratin | 5,0 |
| Panthenol | | | 0.8 |
| Aloe Vera Gel | | | 2,0 |
| Wasser | | | ad 100 |
| | | | |
| pH - Wert | | 3 - 4 | |

### 5. 2-in-1 Shampoo

| Bestandteil | Chem: Bezeichnung | INCI - Bezeichnung | Gew.% |
|---|---|---|---|
| TEXAPON^{®} N 70 (Cognis) | Natriumlaurylethersulfat mit 2 Mol EO (ca. 70%) | Sodium Laureth Sulfate | 12,0 |
| DEHYTON^{®} PK 45 (Cognis) | Fettsäure-amid- Derivat mit Betain-struktur (ca. 45%) | Cocamidopropyl Betaine | 2,5 |
| PLANTACARE^{®} 818 UP (Cognis) | C 8 - C 16 Fettalkoholglycosid (ca. 50%) | Coco Glucoside | 3,0 |
| LAMESOFT^{®} PO 65 (Cognis) | Coco-Glucoside (and) Glyceryl Oleate | Coco-Glucoside (and) Glyceryl Oleate | 3,0 |
| COSMEDIA^{®} GUAR C 261 N (Cognis) | Guarhydroxypropyltrimethyl- ammonium Chlorid | Guar Hydroxypropyl Trimonium Chloride | 0,3 |
| EUPERLAN^{®} PK 1200 (Cognis) | Flüssige Dispersion von perlglanzgebenden Substanzen und Hilfsmittel | Coco-Glucoside (and) Glykol Distearate (and) Glycerin | 5,0 |
| Glycerylstearat Teilchengröße 0,5 mm | | | 2,0 |
| Natriumchlorid | | | 1,2 |
| Euxyl^{®} K 400 (Schülke & Mayr) | | | 0,1 |
| Wasser | | | Ad 100 |
| pH - Wert | | 5,5 | |
| | | | |
| Viskosität (mPas), Brookfield RFT, 23°C, Sp.4, 10 Upm | | 6300 | |

### 6. Perlglänzendes Pflegeshampoo

| Bestandteil | Chem. Bezeichnung | INCI - Bezeichnung | Gew.% |
|---|---|---|---|
| TEXAPON^{®} NSO (Cognis) | Natriumlaurylethersulfat (ca. 28%) | Sodium Laureth Sulfate | 29,0 |
| PLANTACARE^{®} 818 UP (Cognis) | C 8 - C 16 Fettalkoholglycosid (ca. 50%) | Coco Glucoside | 5,0 |
| TEXAPON^{®} SB 3 KC (Cognis) | Sulfobernsteinsäurehalbester auf Basis eines Alkylpolyglycolethers, Di-Na-Salz (ca. 40%) | Disodium Laureth Sulfosuccinate | 3,8 |
| HYDAGEN^{®} HSP (Cognis) | | Trimethylolpropane- Hydroxymethylstearate- Ether | 0,5 |
| EUPERLAN^{®} PK 3000 AM (Cognis) | Flüssige Dispersion von perlglanzgebenden Substanzen und Amphotensid | Glycol Distearate (and) Laureth-4 (and) Cocamidopropyl Betaine | 3,0 |
| Glycerylstearate Teilchengröße 0,25 mm | | | 2,0 |
| NaCl | | | Ad 100 |
| Wasser | | | |
| | | | |
| pH - Wert | | 5,5 | |
| Viskosität (mPas), Brook.RVF, 23° C, Spindel 4, 10 Upm | | 4100 | |

### 7. Reinigungsmilch

| Bestandteil | Chem. Bezeichnung | INCI Bezeichnung | Gew.% |
|---|---|---|---|
| EMULGADE^{®} SE (Cognis) | Gemisch von Partialglyceriden, Fettalkoholen, Wachsestern und ethoxylierten Fettalkoholen | Glyceryl Stearate (and) Ceteareth20 (and) Ceteareth12 (and) Cetearyl Alcohol (and) Cetyl Palmitate | 6,0 |
| EUTANOL^{®} G (Cognis) | 2-Octyldodecanol (Guerbet-Alkohol) | Octyldodecanol | 7,0 |
| Behensäure (Teilchengröße 1,5 mm) | | | 1,0 |
| CETIOL^{®} 868 (Cognis) | Isooctylstearat | Octyl Stearate | 8,0 |
| Glycerin 86 % | | | 3,0 |
| Carbopol^{®} 981 (Goodrich) | | Carbomer / 2 % Quellung | 10,0 |
| NaOH 10 % | | | 0,8 |
| Wasser | | | ad 100 |
| | | | |
| Viskosität (mPas), Brookfield RVF, 23°C, Spindel.5, | | 10 | |
| Upm | | 8.000 | |

### 8. Hautemulsionen (O/W)

| | | |
|---|---|---|
| Gluadin Almond | 0,3 | 0,3 |
| Emulgade^{®}SE⁵⁹ | 8,0 | 8,0 |
| Cutina^{®}MD-A⁶⁰ | 1,5 | 1,5 |
| Cetyl-/Stearylalkohol | 1,5 | 1,5 |
| Myritol^{®}318⁶¹ | 10,0 | 10,0 |
| 2-Ethylhexyl-Stearat | 5,0 | 5,0 |
| Glycerylstearat Teilchengröße 0,25 mm | 1,0 | 0,5 |
| Dimethylpolysiloxan (350 at) | 1,0 | 1,0 |
| Controx^{®}KS⁶² | 0,05 | 0,05 |
| PHB-Propylester | 0,2 | 0,2 |
| PHB-Methylester | 0,2 | 0,2 |
| 1,2-Propylenglycol | 3,0 | 3,0 |
| Hydagen^{®}CMF | 3,0 | 8,0 |
| Cu-Gluconat | 0,04 | 0,10 |
| Wasser (NaOH bis pH = 5) | ad 100 | ad 100 |

| | | |
|---|---|---|
| ⁵⁹ Gemisch aus: Glyceryl Stearate, Ceteareth-20, Ceteareth-12, Cetearyl Alcohol und Cetylpalmitat (COGNIS) ⁶⁰ Glyceryl Stearate (COGNIS) ⁶¹ Caprylic/Capric-Triglyceride (COGNIS) ⁶² Tocopherol und Hydrogenated Tallow Glycerides Citrate (COGNIS) | | |

## Patentansprüche

1. Verwendung von gesättigten oder ungesättigten Fettsäuren mit mindestens 18 C-Atomen, deren Mono- oder Diglyceriden oder Mischungen davon, in Teilchenform mit einem mittleren Teilchendurchmesser im Bereich von 0,05 bis 2 mm, als pflegende Peelingkörper in kosmetischen Mitteln.

2. Verwendung nach Anspruch 1, **dadurch gekennzeichnet, dass** die Teilchen einen mittleren Teilchendurchmesser im Bereich von 0,1 bis 1 mm aufweisen.

3. Verwendung nach Anspruch 1 oder 2, **dadurch gekennzeichnet, dass** die Teilchen im Temperaturbereich von 20 bis 50 °C schmelzen oder erweichen.

4. Verwendung nach einem der Ansprüche 1 bis 3, **dadurch gekennzeichnet, dass** die gesättigten Fettsäuren mindestens 18 C-Atome, die ungesättigten Fettsäuren mindestens 20 C-Atome und die als Mono- oder Diglyceride vorliegenden Fettsäuren mindestens 22 C-Atome aufweisen.

5. Verwendung nach einem der Ansprüche 1 bis 4, **dadurch gekennzeichnet, dass** die kosmetischen Mittel zur kosmetischen Behandlung, insbesondere Reinigung, Peeling und/oder Pflege, der menschlichen Haut dienen.

6. Verwendung nach einem der Ansprüche 1 bis 5, **dadurch gekennzeichnet, dass** die kosmetischen Mittel in Form von tensidhaltigen wässrigen Lösungen oder in Form von Emulsionen vorliegen.

7. Kosmetisches Mittel, enthaltend, bezogen auf das gesamte Mittel, 0,05 bis 10 Gew.-% an gesättigten oder ungesättigten Fettsäuren mit mindestens 18 C-Atomen, deren Mono- oder Diglyceriden oder Mischungen davon, in Teilchenform mit einem mittleren Teilchendurchmesser im Bereich von 0,05 bis 2 mm.

8. Mittel nach Anspruch 7, **dadurch gekennzeichnet, dass** die Teilchen einen mittleren Teilchendurchmesser im Bereich von 0,1 bis 1 mm aufweisen.

9. Mittel nach Anspruch 7 oder 8, **dadurch gekennzeichnet, dass** die Teilchen im Temperaturbereich von 20 bis 50 °C schmelzen oder erweichen.

10. Mittel nach einem der Ansprüche 7 bis 9, **dadurch gekennzeichnet, dass** die kosmetischen Mittel in Form von tensidhaltigen wässrigen Lösungen oder in Form von Emulsionen vorliegen.

11. Mittel nach einem der Ansprüche 7 bis 10, **dadurch gekennzeichnet, dass** es sich um ein kosmetisches Mittel zur Behandlung, insbesondere Reinigung, Peeling und/oder Pflege, der menschlichen Haut handelt.

12. Mittel nach einem der Ansprüche 7 bis 11, **dadurch gekennzeichnet, dass** es sich um ein Rinse-Off-Mittel handelt.

13. Mittel nach einem der Ansprüche 7 bis 12, **dadurch gekennzeichnet, dass** es in Form einer Seife, Syndet-Seife, Halbsyndet-Seife, Creme, Lotion, Lösung, Emulsion, Schüttelmixtur oder eines Gels vorliegt.

14. Mittel nach einem der Ansprüche 7 bis 13, **dadurch gekennzeichnet, dass** es, bezogen auf das gesamte Mittel, 0,1 bis 40 Gew.-% Tenside und/oder 0,1 bis 25 Gew.-% Emulgatoren enthält.

15. Teilchen mit einem mittleren Teilchendurchmesser im Bereich von 0,05 bis 2 mm, enthaltend bezogen auf die Teilchen, mindestens 50 Gew.-% gesättigte oder ungesättigte Fettsäuren mit mindestens 18 C-Atomen, deren Mono- oder Diglyceride oder Mischungen davon.

## Claims

1. The use of saturated or unsaturated fatty acids with at least 18 C atoms, their mono- or di-glycerides or mixtures thereof, in particle form with an average particle diameter in the range from 0.05 to 2 mm, as a peeling care body in cosmetic agents.

2. The use according to claim 1, **characterized in that** the particles have an average particle diameter in the range from 0.1 to 1 mm.

3. The use according to claim 1 or 2, **characterized in that** the particles melt or soften in a temperature range from 20 to 50°C.

4. The use according to any of claims 1 to 3, **characterized in that** the saturated fatty acids have at least 18 C atoms, the unsaturated fatty acids have at least 20 C atoms and the fatty acids existing as mono- or di-glycerides have at least 22 C atoms.

5. The use according to any of claims 1 to 4, **characterized in that** the cosmetic agents are used for cosmetic treatment, in particular cleansing, peeling and/or care of the human skin.

6. The use according to any of claims 1 to 5, **characterized in that** the cosmetic agents exist in the form of surfactant-containing aqueous solutions or in the form of emulsions.

7. A cosmetic agent, containing, based on the total agent, 0.05 to 10% by weight of saturated or unsaturated fatty acids with at least 18 C atoms, their mono- or di-glycerides or mixtures thereof, in the form of particles with an average particle diameter in the range from 0.05 to 2 mm.

8. The agent according to claim 7, **characterized in that,** the particles have an average particle diameter in the range from 0.1 to 1 mm.

9. The agent according to claim 7 or 8, **characterized in that,** the particles melt or soften in the temperature range from 20 to 50°C.

10. The agent according to any of claims 7 to 9, **characterized in that** the cosmetic agents exist in the form of surfactant-containing aqueous solutions or in the form of emulsions.

11. The agent according to any of claims 7 to 10, **characterized in that** it is a cosmetic agent for treatment, in particular cleansing, peeling and/or care of the human skin.

12. The agent according to any of claims 7 to 11, **characterized in that** it is a rinse-off agent.

13. The agent according to any of claims 7 to 12, **characterized in that** it exists in the form of a soap, a syndet soap, a half-syndet soap, a cream, a lotion, a solution, an emulsion, a shaking mixture or a gel.

14. The agent according to any of claims 7 to 13, **characterized in that** it contains, based on the total agent, 0.1 to 40% by weight of surfactants and/or 0.1 to 25% by weight of emulsifiers.

15. Particles with an average particle diameter in the range from 0.05 to 2 mm, containing, based on the particles, at least 50% by weight of saturated or unsaturated fatty acids with at least 18 C atoms, their mono- or di-glycerides or mixtures thereof.

## Revendications

1. Utilisation d'acides gras saturés ou insaturés contenant au moins 18 atomes de carbone, de leurs mono- ou diglycérides ou encore de leurs mélanges, sous forme particulaire avec un diamètre moyen de particule dans la plage de 0,05 à 2 mm, à titre d'exfoliant de soins dans des agents cosmétiques.

2. Utilisation selon la revendication 1, **caractérisée en ce que** les particules présentent un diamètre moyen de particule dans la plage de 0,1 à 1 mm.

3. Utilisation selon la revendication 1 ou 2, **caractérisée en ce que** les particules ont un point de fusion ou se ramollissent dans la plage de température de 20 à 50 °C.

4. Utilisation selon l'une quelconque des revendications 1 à 3, **caractérisée en ce que** les acides gras saturés présentent au moins 18 atomes de carbone, les acides gras insaturés présentent au moins 20 atomes de carbone et les acides gras qui se présentent sous la forme de monoglycérides ou de diglycérides présentent au moins 22 atomes de carbone.

5. Utilisation selon l'une quelconque des revendications 1 à 4, **caractérisée en ce que** les agents cosmétiques servent au traitement cosmétique, en particulier au nettoyage, à l'exfoliation et/ou soins de la peau humaine.

6. Utilisation selon l'une quelconque des revendications 1 à 5, **caractérisée en ce que** les agents cosmétiques sont présents sous la forme de solutions aqueuses tensioactives ou sous la forme d'émulsions.

7. Agent cosmétique contenant, rapportés à l'agent total, à concurrence de 0,05 à 10 % en poids, des acides gras saturés ou insaturés contenant au moins 18 atomes de carbone, de leurs mono- ou diglycérides ou encore de leurs mélanges, sous forme particulaire avec un diamètre moyen de particule dans la plage de 0,05 à 2 mm.

8. Agent selon la revendication 7, **caractérisé en ce que** les particules présentent un diamètre moyen de particule dans la plage de 0,1 à 1 mm.

9. Agent selon la revendication 7 ou 8, **caractérisé en ce que** les particules ont un point de fusion ou se ramollissent dans la plage de température de 20 à 50 °C.

10. Agent selon l'une quelconque des revendications 7 à 9, **caractérisé en ce que** les agents cosmétiques se présentent sous la forme de solutions aqueuses tensioactives ou sous la forme d'émulsions.

11. Agent selon l'une quelconque des revendications 7 à 10, **caractérisé en ce qu'**il s'agit d'un agent cosmétique destiné au traitement, en particulier le nettoyage, l'exfoliation et/ou les soins de la peau humaine.

12. Agent selon l'une quelconque des revendications 7 à 11, **caractérisé en ce qu'**il s'agit d'un agent qui s'élimine par rinçage.

13. Agent selon l'une quelconque des revendications 7 à 12, **caractérisé en ce qu'**il est présent sous la forme d'un savon, d'un savon syndet, d'un savon semi-syndet, d'une crème, d'une lotion, d'une solution, d'une émulsion, d'un mélange à agiter ou d'un gel.

14. Agent selon l'une quelconque des revendications 7 à 13, **caractérisé en ce qu'**il contient, rapportés à l'agent total, des agents tensioactifs à concurrence de 0,1 à 40 % en poids et/ou des émulsifiants à concurrence de 0,1 à 25 % en poids.

15. Particules avec un diamètre moyen de particule dans la plage de 0,05 à 2 mm contenant, rapportés aux particules, à concurrence d'au moins 50 % en poids, des acides gras saturés ou insaturés contenant au moins 18 atomes de carbone, leurs mono- ou diglycérides ou encore leurs mélanges.
